(19) Europäisches Patentamt

European Patent Office

Office européen des brevets

(11) Veröffentlichungsnummer: **0 363 581 B1**

(12) **EUROPÄISCHE PATENTSCHRIFT**

(45) Veröffentlichungstag der Patentschrift: **02.12.92**

(21) Anmeldenummer: **89113753.1**

(22) Anmeldetag: **26.07.89**

(51) Int. Cl.⁵: **A61M 25/01**, A61M 25/08

(54) **Anordnung zur Drainage von Körperhohlräumen.**

(30) Priorität: **17.09.88 DE 3831652**

(43) Veröffentlichungstag der Anmeldung:
**18.04.90 Patentblatt  90/16**

(45) Bekanntmachung des Hinweises auf die
Patenterteilung:
**02.12.92 Patentblatt  92/49**

(84) Benannte Vertragsstaaten:
**AT DE FR GB IT**

(56) Entgegenhaltungen:
**DE-A- 3 339 179**
**DE-A- 3 446 940**
**DE-A- 3 714 839**

(73) Patentinhaber: **WILLY RÜSCH AG**
**Willy-Rüsch-Strasse 4-10**
**W-7053 Kernen/Rommelshausen(DE)**

(72) Erfinder: **Rüsch, Heinz**
**Nachtigallenweg 6**
**W-7050 Waiblingen(DE)**

(74) Vertreter: **KOHLER SCHMID + PARTNER Patentanwälte**
**Ruppmannstrasse 27**
**W-7000 Stuttgart 80(DE)**

Rank Xerox (UK) Business Services

## Beschreibung

Die Erfindung betrifft eine Anordnung zur Drainage von Körperhohlräumen, insbesondere des Nierenhohlsystems, bestehend aus einem biegsamen Drainagerohr mit mindestens einem gekrümmten Ende, einem zum Vorschieben des Drainagerohres in den Körperhohlraum dienenden, Pusher genannten, schlauchförmigen Hilfsinstrument, dessen proximales Ende mit dem distalen Ende des Drainagerohres lösbar verbunden ist, und einem das Drainagerohr und den Pusher durchsetzenden Führungsdraht.

Solche Anordnungen sind in vielfältigen Ausführungsformen bekannt. Das Drainagerohr dient dazu, verengte, natürliche oder künstliche Körperkanäle offenzuhalten und den Transport von Körperflüssigkeiten von Hohlorgan zu Hohlorgan oder auch von Hohlorganen oder aus Gewebeschichten nach außen zu gewährleisten. Dabei dienen der Pusher und der Führungsdraht zum Einführen des Drainagerohres in den offen zu haltenden Körperkanal.

Ein besonderes Anwendungsgebiet ist die Drainage des Nierenhohlsystems. Dabei ist es erforderlich, das Drainagerohr in den Nierenbecken und Harnblase verbindenden Harnleiter einzubringen. Dabei hat sich ein Drainagerohr besonders bewährt, das wenigstens an einem Ende vorzugsweise aber an beiden Enden ähnlich einem "J" gekrümmt oder sogar wie ein Schweineschwanz eingerollt ist. An beiden Enden gekrümmte Drainagerohre sind auch unter der Bezeichnung "Double-J" oder ,"Pigtail" bekannt. Der Führungsdraht dient dabei dazu, die gekrümmten Enden des Drainagerohres zu strecken. Eine solche Anordnung wird von der Anmelderin unter der Bezeichnung "Integral-Uretersplint-Set" hergestellt und vertrieben, siehe z.B. den Katalog der Anmelderin "Urologie II/6" aus dem Jahre 1986.

Die gekrümmten Enden des Drainagerohres haben den Zweck, das Drainagerohr mit seinen Enden im Nierenbecken bzw. der Harnblase zu verankern und dadurch ein Wandern des Drainagerohres im Harnleiter zu verhindern. Es ist ohne weiteres ersichtlich, daß das Einführen des Drainagerohres nicht nur dessen axiales Verschieben erfordert, sondern auch gegebenenfalls sein Drehen erfordert, um die gekrümmten Enden in den Hohlorganen in die für einen einwandfreien Sitz richtige Lage zu bringen. Dabei werden die zum Verschieben und Drehen des Drainagerohres erforderlichen Kräfte von dem Pusher übertragen, der zu diesem Zweck mit dem Drainagerohr fest verbunden ist.

Nach dem Einsetzen des Drainagerohres müssen der Führungsdraht und der Pusher entfernt werden. Während der Führungsdraht einfach aus dem Drainagerohr und dem ebenfalls rohrförmigen Pusher herausgezogen werden kann, muß die Verbindung zwischen dem Drainagerohr und dem Pusher getrennt werden. Bei bekannten Anordnungen (siehe z.B. DE-A-3 339 179) besteht eine feste Verbindung zwischen dem Pusher und dem Drainagerohr, die mittels eines speziellen Hilfsinstrumentes getrennt werden muß. Das Durchführen einer solchen Trennung ist äußerst schwierig, da das Trenninstrument entweder durch das zum Einführen des Drainagerohres verwendete Cystoskop oder aber auch direkt durch die Harnröhre eingeführt werden muß. Bei einer anderen bekannten Anordnung (siehe DE-A-3 446 940) ist der Pusher mit dem Drainagerohr mechanisch verkeilt, jedoch ist auch eine solche Anordnung unbefriedigend, weil sich die Keilverbindung schon beim Einbringen des Drainagerohres unkontrolliert lockern oder aber auch so fest werden kann, daß eine spätere Trennung nicht mehr ohne Schwierigkeiten möglich ist.

Demgemäß liegt der Erfindung die Aufgabe zugrunde, bei einer Anordnung der eingangs beschriebenen Art den Pusher mit dem Drainagerohr so zu verbinden, daß eine einwandfreie Übertragung der zum Einlegen des Drainagerohres erforderlichen Kräfte gewährleistet ist, jedoch die Verbindung zwischen Drainagerohr und Pusher ohne weiteres leicht trennbar ist.

Diese Aufgabe wird nach der Erfindung dadurch gelöst, daß die miteinander formschlüssig verbundenen Enden des Drainagerohres und des Pushers mit zueinander komplementären Teilen eines Schlosses versehen sind, die einander überlappende Abschnitte aufweisen, die ihrerseits mit aneinander anliegenden, zur Übertragung axialer Zugkräfte und eines Drehmomentes geeigneten Flächenabschnitten versehen sind und von dem sie durchsetzenden Führungsdraht in Eingriff gehalten werden.

Bei der erfindungsgemäßen Anordnung wird mit Hilfe eines Schlosses, dessen Teile durch den Führungsdraht verriegelt sind, eine einwandfreie formschlüssige Verbindung zwischen dem Pusher und dem Drainagerohr geschaffen, die eine einwandfreie Übertragung aller Axial- und Drehbewegungen vom Pusher auf das Drainagerohr gewährleistet, die jedoch nach Herausziehen des Führungsdrahtes gelöst wird, weil dann die beiden Teile des Schlosses nicht mehr zusammengehalten werden, sondern die Tendenz haben, sich von allein zu trennen, weil das bisher durch den Führungsdraht gestreckt gehaltene, von der Katheterspitze abgewandte Ende des Drainagerohres bestrebt ist, seine gekrümmte Lage einzunehmen und sich dadurch vom Ende des Pushers trennt. Daher ist es vorteilhaft, wenn die zur Kraftübertragung dienenden Flächen an der Außenseite des gekrümmten Endes des Drainagerohres angeordnet sind.

Bei einer bevorzugten Ausführungsform der Erfindung weist jedes Teil des Schlosses im Abstand von seinem Ende eine Quernut und der die Quernut begrenzende Abschnitt an seinem Umfang eine zum Nutgrund parallele Abflachung auf. Bei dieser Anordnung nimmt jedes Teil in seiner Quernut den die Quernut begrenzenden Abschnitt des anderen Teiles auf und es kommt jeweils der die Quernut begrenzende Abschnitt eines Teiles mit seiner Abflachung am Nutgrund des anderen Teiles zur Anlage. Die in die Quernut eingreifenden Abschnitte des anderen Teiles gewährleisten mit ihren quer zur Achse des Drainagerohres und des Pushers gerichteten Flächen eine einwandfreie Übertragung von Axialkräften, während der Formschluß zwischen den parallelen Abflachungen an den die Quernut begrenzenden Abschnitten und dem jeweils benachbarten Nutgrund auch eine einwandfreie Übertragung von Drehmomenten gewährleistet. Dabei ist es für einen einwandfreien Zusammenhalt der Schloßteile besonders vorteilhaft, wenn die Quernut die Bohrung des Teiles schneidet, weil dann auch der jeweils die Quernut begrenzende Abschnitt eine geschlossene Bohrung aufweist, mit der dieser Abschnitt auf dem Führungsdraht einwandfrei zentriert ist.

Die Teile des Schlosses können von besonderen Teilen gebildet werden, die in das Drainagerohr und/oder in den Pusher eingesetzt sind, sie können aber auch unmittelbar an das Drainagerohr und/oder an den Pusher angeformt sein. Finden eingesetzte Teile Verwendung, können sie aus jedem ausreichend festen, medizinisch unbedenklichen Material bestehen, insbesondere aus Metall oder Kunststoff.

In einer weiteren Ausgestaltung der Erfindung ist das Schloß von einer Hülse umgeben.

Dies hat den Vorteil, daß das mit dem Pusher verbundene Drainagerohr auf einen schon gelegten Spiralmandrin koaxial aufgefädelt werden kann. Die Spitze des Drainagerohres weist dazu einen Durchbruch auf, über den das Drainagerohr auf den Spiralmandrin aufgefädelt werden kann. Sobald der Spiralmandrin die Bohrungsöffnungen des Schlosses durchstoßen hat, kann die Hülse abgenommen werden. Das Schloß wird vom Spiralmandrin zentriert zusammengehalten.

In zusätzlicher Ausgestaltung der Erfindung ist die Hülse in zwei Hülsenhälften aufklappbar.

Dies hat den Vorteil, daß die komplementären Teile des Schlosses fest aneinandergepreßt werden können und die Hülse einen axial unverrückbaren Preßsitz am Ende des Pushers und des Drainagerohres aufweist. Sobald der Spiralmandrin die Teile des Schlosses zusammenhält, kann durch ein Auseinanderklappen der Hülsenhälften die Hülse vom Ende des Drainagerohres und dem Pusher einfach abgenommen werden.

Nach Beendigung der indizierten Drainage muß das Drainagerohr wieder aus den Körperhohlräumen entfernt werden. Dieses Entfernen erfolgt nach bekannten Methoden, insbesondere mittels eines endoskopischen Eingriffes. Bei kurzer Verweilzeit hat es sich bewährt, an dem Drainagerohr dünne Fäden zu befestigen, die aus dem Körperkanal, z.B. aus der Harnröhre, heraushängen, so daß das Entfernen des Drainagerohres durch Herausziehen an den Fadenenden erfolgen kann. Bei der erfindungsgemäßen Anordnung besteht die Möglichkeit, an dem Schloßteil des Drainagerohres wenigstens einen Faden anzubringen, der so lang ist, daß er bei eingelegtem Drainagerohr aus dem Körperhohlraum herausragt und dadurch das Herausziehen des Drainagerohres ermöglicht.

Die Erfindung wird im folgenden anhand der in der Zeichnung dargestellten Ausführungsbeispiele näher beschrieben und erläutert. Die der Beschreibung und der Zeichnung zu entnehmenden Merkmale können bei anderen Ausführungsformen der Erfindung einzeln für sich oder zu mehreren in beliebiger Kombination Anwendung finden.

Es zeigen:

Fig. 1     eine nach der Erfindung ausgebildete Anordnung zur Drainage von Körperhohlräumen;

Fig. 2     das Drainagerohr der Anordnung nach Fig. 1 nach Herausziehen des Führungsdrahtes und Abtrennen des Pushers;

Fig. 3     einen Längsschnitt durch den Abschnitt III der Anordnung nach Fig. 1 in vergrößertem Maßstab;

Fig. 4     einen Schnitt längs der Linie IV-IV durch die Anordnung nach Fig. 3;

Fig. 5     einen Schnitt ähnlich Fig. 3 durch eine weitere Ausführungsform der Erfindung;

Fig. 6     einen Längsschnitt durch eine Ausführungsform eines erfindungsgemäßen Schlosses mit einer Hülse;

Fig. 7     eine perspektivische Darstellung eines Abschnitts eines erfindungsgemäßen Schlosses.

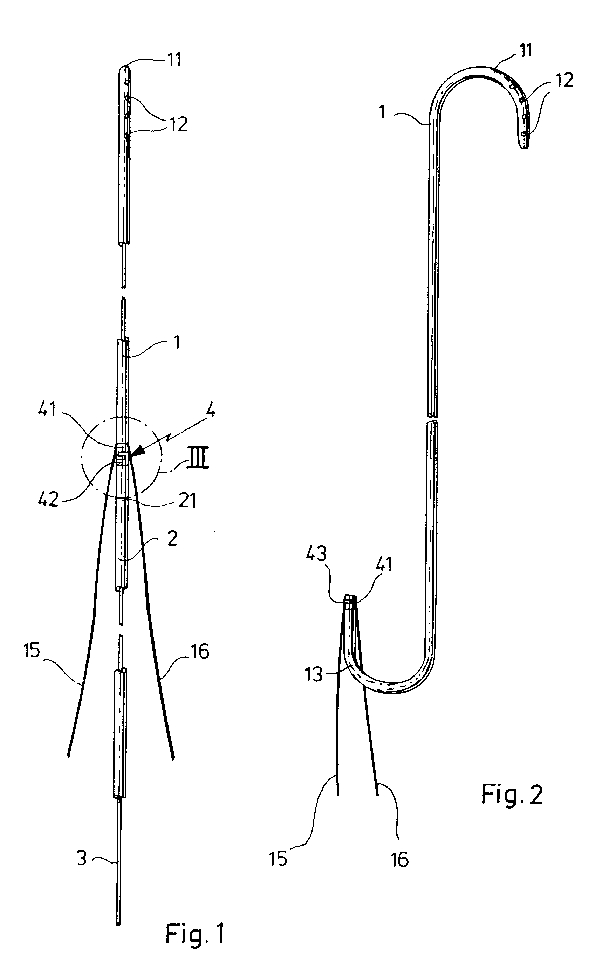

Die in Fig. 1 dargestellte Anordnung umfaßt ein Drainagerohr 1, einen Pusher 2 und ein das Drainagerohr 1 und den Pusher 2 durchsetzenden Führungsdraht 3. Das Drainagerohr 1 und der Pusher 2 bestehen aus einem biegsamen Schlauch, der z.B. aus einem physiologisch verträglichen Kunststoff hergestellt ist. Das Drainagerohr 1 ist an seinem proximalen Ende 11, der Katheterspitze, geschlossen, jedoch im Bereich dieses Endes mit seine Wandungen durchsetzenden Drainageöffnungen 12 versehen. Die Drainageöffnungen 12 können längs des ganzen Drainagerohres 1 angeordnet sein. In der Fig. 2 sind die Drainageöffnungen 12 nur im Bereich des Endes 11 eingezeichnet. Ferner kann die Spitze des Endes 11 des Drainagerohres 1 einen Durchbruch aufweisen, über den das Drainagerohr 1 auf einen schon gelegten Spiralmandrin koaxial aufgefädelt werden kann. An seinem distalen Ende 13, dem der Katheterspitze abgewandten Ende, weist das Drainagerohr 1 das Teil 41 eines Schlosses 4 auf, das mit einem zweiten, komplementären Teil 42 zusammen wirkt, das am proximalen Ende 21 des Pushers 2, dem Ende, das dem Drainagerohr 1 zugewandt ist, angebracht ist. Der in den Pusher 2 und das Drainagerohr 1 eingeführte Führungsdraht 3 gibt der Anordnung die Steifigkeit, die erforderlich ist, um das Drainagerohr 1 in einen Körperkanal einzuschieben.

Bei dem dargestellten Ausführungsbeispiel handelt es sich um ein Drainagerohr zur Verbindung des Nierenbeckens mit der Harnblase, der in den Harnleiter einzuführen ist. Das Einführen erfolgt in bekannter Weise mittels eines Cystoskops. Dabei wird das Drainagerohr 1 mittels des aus dem Cystoskop herausragenden Pushers 2 vorgeschoben, bis er die gewünschte Stellung erreicht hat.

Der mit dem Drainagerohr 1 mittels des Schlosses 4 fest verbundene Pusher 2 bietet auch die Möglichkeit, das Drainagerohr 1 bei Bedarf wieder leicht zurückzuziehen und auch zu drehen, um es in die richtige Lage zu bringen. Nachdem das Drainagerohr 1 in die gewünschte Stellung gebracht worden ist, müssen der Pusher 2 und der Führungsdraht 3 entfernt werden. Der Führungsdraht 3 läßt sich ohne weiteres aus dem Pusher 2 und dem Drainagerohr 1 herausziehen. Nach dem Herausziehen des Führungsdrahtes 3 löst sich das Schloß 4, so daß dann auch der Pusher 2 entfernt werden kann, während das Drainagerohr 1 an seiner Stelle verbleibt. Die Enden 11 und 13 des Drainagerohres 1 sind so vorgespannt, daß sie sich krümmen und eine Art doppeltes J bilden. Deshalb werden solche Drainagerohre auch häufig "Double-J" genannt. Die hakenförmigen Krümmungen an den Enden des Drainagerohres 1 dienen zu dessen Sicherung gegen ein Wandern im Harnleiter.

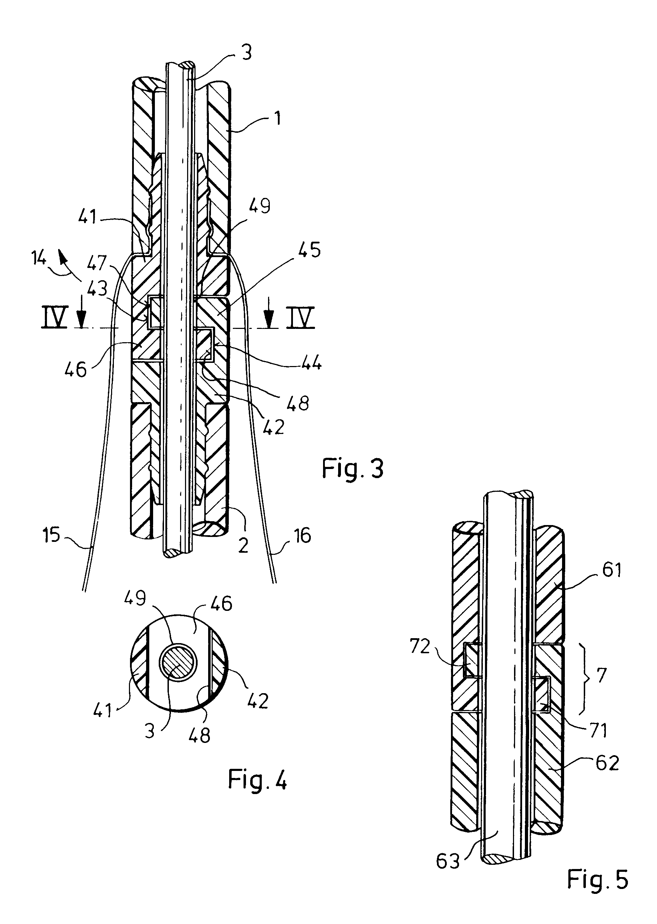

Wie aus den Fig. 3 und 4 näher ersichtlich, sind die Teile 41 und 42 des Schlosses in das jeweilige Ende des Drainagerohres 1 bzw. des Pushers 2 eingesetzt. Sie können aus Metall, aber auch aus Kunststoff bestehen. Jedes dieser Teile weist im Abstand von seinem Ende eine Quernut 43 bzw. 44 auf, in die der die Quernut nach außen begrenzende Abschnitt 45 bzw. 46 des jeweils anderen Teiles eingreift. Dabei ist jeder dieser Abschnitte mit einer Abflachung 47 bzw. 48 versehen, die parallel zum Nutgrund verläuft und mit der er jeweils am Nutgrund des anderen Teiles anliegt. Die Quernuten 43, 44 sind so tief, daß sie die Bohrung 49 des jeweiligen Teiles schneiden, so daß also auch die äußeren, die Quernuten 43, 44 begrenzenden Abschnitte 45, 46 der Teile 41, 42 eine Bohrung 49 für den Führungsdraht 3 aufweisen.

Wenn die beiden komplementären Teile 41, 42 des Schlosses 4 ineinander greifen und von dem Führungsdraht 3 durchsetzt werden, sind sie formschlüssig fest miteinander verbunden. Dabei erlauben die quer zu ihrer Achsrichtung gerichteten Flächen der Quernuten 43, 44 und der die Quernuten begrenzenden Abschnitte 45, 46 die Übertragung von axialen Kräften, während die an den Nutgründen anliegenden Abflachungen 47, 48 der in die Quernuten eingreifenden Abschnitte 45, 46 die Übertragung von Drehmomenten gestatten. Wird jedoch nach Einlegen des Drainagerohres 1 der Führungsdraht 3 aus der Anordnung herausgezogen, läßt sich die formschlüssige Verbindung zwischen den beiden Teilen 41, 42 des Schlosses 4 leicht trennen. Dabei ist die Quernut 43 in dem am Drainagerohr 1 angebrachten Teil 41 entgegengesetzt zu der durch den Pfeil 14 dargestellten Bewegung gerichtet, die das Ende 13 des Drainagerohres 1 auszuführen bestrebt ist, um die in Fig. 2 dargestellte, gekrümmte Lage einzunehmen. Dies bedeutet, daß die in dem Teil 41 vorhandene Quernut 43 zur Außenseite des gekrümmten Ende des Drainagerohres 1 gerichtet ist. So weit ein gewisser Reibungsschluß zwischen den Teilen 41, 42 diese Bewegung hindert, kann dieser Reibungsschluß durch leichtes Drehen des Pushers 2 um seine Achse gelöst werden.

Fig. 5 zeigt eine alternative Ausführungsform der Erfindung, bei der das Schloß 7 die soeben beschriebene Konfiguration aufweist, seine Teile 71. 72 jedoch unmittelbar an das Ende des Drainagerohres 61 bzw. des Pushers 62 angeformt sind. Auch hier werden die Teile 71, 72 in der soeben beschriebenen Weise formschlüssig zusammengehalten, so lange der Führungsdraht 63 die das Schloß 7 bildenden Abschnitte des Drainagerohres 61 und des Pushers 62 durchsetzt.

Die Entfernung des Drainagerohres erfolgt gewöhnlich mittels eines endoskopischen Eingriffes. Wenn jedoch die voraussichtliche Verweildauer des Drainagerohres nur kurz ist, insbesondere nicht mehr als fünf Tage beträgt, ist es vertretbar, an dem Ende des Drainagerohres dünne Fäden zu befestigen, die so lang sind, daß sie aus dem Körper, also insbesondere aus der Harnröhre, heraushängen. In diesem Fall kann ein endoskopischer Eingriff vermieden und das Drainagerohr einfach durch Herausziehen mittels der Fäden erfolgen. Bei der Ausführungsform nach den Fig. 1 bis 4 sind solche Fäden 15, 16 zusammen mit dem Teil 41 des Schlosses 4 am Drainagerohr 1 befestigt.

Es versteht sich, daß die Erfindung nicht auf die dargestellten Ausführungsformen beschränkt ist, sondern Abweichungen davon möglich sind, ohne den Rahmen der Erfindung zu verlassen. Insbesondere kann ein nach der Erfindung das Drainagerohr und den Pusher verbindendes Schloß an Drainagerohren beliebiger Ausbildung angebracht werden, also insbesondere auch an solchen Katheterrohren, die nur ein gekrümmtes Ende aufweisen oder bei denen die Krümmung anders gestaltet ist, wie es beispielsweise bei den "Pigtail" genannten Drainagerohren der Fall ist. Weiterhin können solche Verbindungen auch bei Drainageanordnungen anderer Art, die nicht für die Urologie bestimmt sind, Anwendung finden. Weiterhin ist die Erfindung nicht auf die spezielle Konfiguration der komplementären Teile des Schlosses beschränkt, sondern es können die Teile z.B. auch mit Schrägflächen versehen sein, die aneinander anliegen und dazu geeignet sind, axiale und/oder radiale Kräfte zu übertragen, so lange sie durch einen Riegel zusammengehalten werden, die jedoch eine leichte Trennung gewährleisten, wenn der Riegel entfernt wird. Dabei braucht als Riegel nicht unbedingt ein ohnehin benötigter Führungsdraht verwendet zu werden, sondern es sind auch Anordnung denkbar, bei denen ein von außen herausziehbarer Draht ausschließlich zum Zweck der Verriegelung der Schloßteile vorgesehen ist.

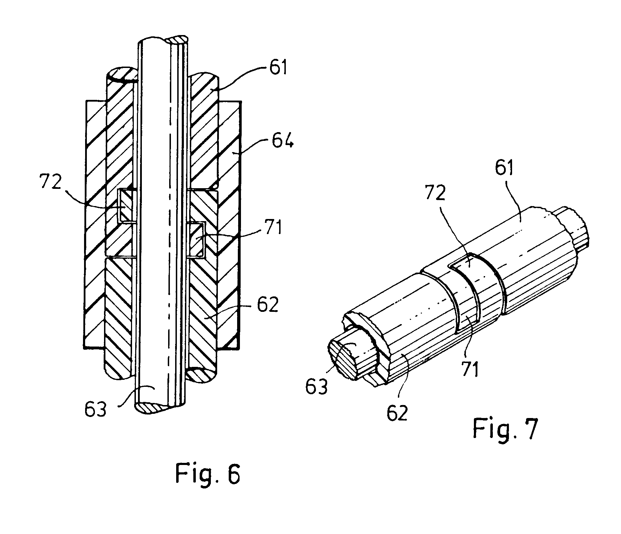

Wird das Drainagerohr 61, wie in Fig. 6 dargestellt, auf einen schon gelegten Führungsdraht 63 (Spiralmandrin) koaxial aufgefädelt, so weist das Drainagerohr 1 an der Spitze, dem Ende 11, einen Durchbruch auf. Solange der Spiralmandrin noch nicht die Teile 71, 72, des Schlosses zusammenhält, wird das Drainagerohr 61 und der Pusher 62 von einer Hülse 64 zusammengehalten, die entweder als Schlauchhülse vor dem Zusammenschluß der Teile 71, 72 des Schlosses über das Pusherende mit dem Teil 72 geschoben wird und nach dem Zusammenschluß der Teile 71, 72 wird die Schlauchhülse über die Teile 71, 72 verschoben oder die Hülse 64 ist in zwei Hülsenhälften aufklappbar.

Fig. 7 zeigt einen Abschnitt der Enden des Drainagerohres 61 und des Pushers 62 in perspektivischer Darstellung. Die Darstellung ist nicht maßstäblich zu verstehen. In der Figur ist gezeigt, wie die Teile 71, 72 formschlüssig ineinandergreifen und wie der Führungsdraht 63 die Teile 71, 72 zusammenhält.

**Patentansprüche**

1. Anordnung zur Drainage von Körperhohlräumen, insbesondere des Nierenhohlsystems, bestehend aus einem biegsamen Drainagerohr (1) mit mindestens einem gekrümmten Ende, einem zum Vorschieben des Drainagerohres in den Körperhohlraum dienenden, Pusher genannten, schlauchförmigen Hilfsinstrument (2), dessen proximales Ende mit dem distalen Ende des Drainagerohres lösbar verbunden ist, und einem das Drainagerohr und den Pusher durchsetzenden Führungsdraht (3), dadurch gekennzeichnet, daß die miteinander formschlüssig verbundenen Enden des Drainagerohres (1) und des Pushers (2) mit zueinander komplementären Teilen (41, 42) eines Schlosses (4) versehen sind, die einander überlappende Abschnitte (45, 46) aufweisen, die ihrerseits mit aneinander anliegenden, zur Übertragung axialer Zugkräfte und eines Drehmomentes geeigneten Flächenabschnitten versehen sind und von dem sie durchsetzenden Führungsdraht (3) in Eingriff gehalten werden.

2. Anordnung nach Anspruch 1, dadurch gekennzeichnet, daß die zur Kraftübertragung dienenden Flächen an der Außenseite des gekrümmten Endes (13) des Drainagerohres (1) angeordnet sind.

3. Anordnung nach Anspruch 1 oder 2, dadurch gekennzeichnet, daß jedes Teil (41, 42) des Schlosses (4) im Abstand von seinem Ende eine Quernut (43, 44) aufweist und der die Quernut begrenzende Abschnitt (45, 46) an seinem Umfang eine zum Nutgrund parallele Abflachung (47, 48) aufweist.

4. Anordnung nach Anspruch 3, dadurch gekennzeichnet, daß die Quernut (43, 44) die Bohrung (49) des Teiles (41, 42) schneidet.

5. Anordnung nach einem der vorhergehenden Ansprüche, dadurch gekennzeichnet, daß wenigstens ein Teil (41, 42) des Schlosses (4) in das Drainagerohr (1) und/oder in den Pusher (2) eingesetzt ist.

6. Anordnung nach einem der vorhergehenden Ansprüche, dadurch gekennzeichnet, daß wenigstens ein Teil des Schlosses (7) unmittelbar an das Drainagerohr (61) und/oder an den Pusher (62) angeformt ist.

7. Anordnung nach einem der vorhergehenden Ansprüche, dadurch gekennzeichnet, daß an dem Schloßteil (41) des Drainagerohrs (1) mindestens ein Faden (15, 16) angebracht ist, der so lang ist, daß er bei eingelegtem Drainagerohr (1) aus dem Körperhohlraum herausragt.

8. Anordnung nach einem der vorhergehenden Ansprüche, dadurch gekennzeichnet, daß das Schloß (4; 7) von einer Hülse (64) umgeben ist.

**9.** Anordnung nach Anspruch 8, dadurch gekennzeichnet, daß die Hülse (64) in zwei Hülsenhälften aufklappbar ist.

## Claims

**1.** Arrangement for draining body cavities, in particular cavities of the renal hollow system, consisting of a flexible drainage pipe (1) with at least one crooked end, an auxiliary tubular instrument (2) called pusher for pushing forward the drainage pipe into the body cavity of which the proximal end is detachably connected with the distal end of the drainage pipe and a guiding wire (3) which runs inside the drainage pipe and the pusher, characterised in that the ends of the drainage pipe (1) and of the pusher (2) which are connected to one another in a form-locking manner are provided with parts (41, 42) of a lock (4) which are complementary to one another and comprise sections (45, 46) which overlap one another and are provided with surface sections which are suitable for transmitting axial tensile forces and a torque and are held in engagement by the guiding wire (3) which runs inside thereof.

**2.** Arrangement according to claim 1, characterised in that the surfaces which serve for transmitting forces are disposed on the outer side of the crooked end (13) of the drainage pipe (1).

**3.** Arrangement according to claim 1 or 2, characterised in that each part (41, 42) of the lock (4) comprises at a distance from its end a transverse groove (43, 44) and the section (45, 46) which delimits the transverse groove comprises at its periphery a flattening (47, 48) which is parallel to the groove bottom.

**4.** Arrangement according to claim 3, characterised in that the transverse groove (43, 44) extends over the bore (49) of the part (41, 42).

**5.** Arrangement according to any one of the preceding claims, characterised in that at least one part (41, 42) of the lock (4) is inserted into the drainage pipe (1) and/or into the pusher (2).

**6.** Arrangement according to any one of the preceding claims, characterised in that at least one part of the lock (7) is formed directly on the drainage pipe (61) and/or on the pusher (62).

**7.** Arrangement according to any one of the preceding claims, characterised in that at least one thread (15, 16) is disposed on the lock part (41) of the drainage pipe (1) which is sufficiently long so that it extends out of the body cavity when the drainage pipe (1) has been inserted.

**8.** Arrangement according to any of the preceding claims, characterised in that the lock (4; 7) is surrounded by a sleeve (64).

**9.** Arrangement according to claim 8, characterised in that the sleeve (64) can be opened into two sleeve parts.

## Revendications

**1.** Agencement pour le drainage de cavités corporelles, en particulier du système creux des reins, constitué par un tube de drainage (1) flexible comportant au moins une extrémité courbée, un instrument auxiliaire (2) en forme de tuyau, dénommé pusher, qui sert à l'avancement du tube de drainage dans la cavité corporelle et dont l'extrémité proximale est reliée de façon détachable à l'extrémité distale du tube de drainage, et un fil de guidage (3) qui traverse le tube de drainage et le pusher, caractérisé en ce que les extrémités, raccordées l'une à l'autre par une liaison par la forme, du tube de drainage (1) et du pusher (2) sont munies de parties réciproquement complémentaires (41, 42) d'un dispositif de fixation (4) et présentant des tronçons (45, 46) qui se chevauchent l'un l'autre et qui sont pourvus de leur côté de segments de faces appuyés l'un sur l'autre, appropriés pour la transmission de forces de traction axiales et d'un couple de rotation et maintenus en prise par le fil de guidage (3) qui les traverse.

**2.** Agencement suivant la revendication 1, caractérisé en ce que les faces servant à la transmission de force sont agencées sur le côté externe de l'extrémité courbée (13) du tube de drainage (1).

**3.** Agencement suivant l'une ou l'autre des revendications 1 et 2, caractérisé en ce que chaque partie (41, 42) du dispositif de fixation (4) présente à distance de son extrémité une rainure transversale (43, 44) et en ce que le tronçon (45, 46) qui délimite la rainure transversale présente à sa périphérie un méplat (47, 48) parallèle au fond de rainure.

**4.** Agencement suivant la revendication 3, caractérisé en ce que la rainure transversale (43, 44) coupe le perçage (49) de la partie (41, 42).

5. Agencement suivant l'une quelconque des revendications précédentes, caractérisé en ce qu'au moins une partie (41, 42) du dispositif de fixation (4) est insérée dans le tube de drainage (1) et/ou dans le pusher (2).

6. Agencement suivant l'une quelconque des revendications précédentes, caractérisé en ce qu'au moins une partie du dispositif de fixation (7) est directement formée sur le tube de drainage (61) et/ou sur le pusher (62).

7. Agencement suivant l'une quelconque des revendications précédentes, caractérisé en ce que sur la partie de dispositif de fixation (41) du tube de drainage (1) est placé au moins un fil (15, 16) dont la longueur est telle qu'il fait saillie de la cavité corporelle lorsque le tube de drainage (1) est mis en place.

8. Agencement suivant l'une quelconque des revendications précédentes, caractérisé en ce que le dispositif de fixation (4; 7) est entouré d'un manchon (64).

9. Agencement suivant la revendication 8, caractérisé en ce que le manchon (64) est repliable en deux demi-manchons.

Fig. 1

Fig. 2

Fig. 3

Fig. 4

Fig. 5

Fig. 6

Fig. 7